# DEMANDE DE BREVET EUROPEEN

(11) **EP 1 559 410 A1**
(43) Date de publication de la demande: **03.08.2005**
(21) Numéro de dépôt: 05290122.0
(22) Date de dépôt: 20.01.2005
(51) Int. Cl.: A61K 7/13

(54) **Composition de teinture des fibres kératiniques contenant une alcool oxydase et un polymère à motif sucre**

(30) Priorité: 28.01.2004 FR 0400777
(71) Demandeur: L'OREAL, 75008 Paris (FR)
(72) Inventeur: Plos, Grégory, Tokyo 158-0097 (JP)
(74) Mandataire: Casalonga, Axel

(57) **Abrégé**

La présente demande concerne une composition pour la teinture des fibres kératiniques, et en particulier les fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins un polymère à motif sucre. Cette demande concerne encore un procédé de teinture des fibres kératiniques qui consiste à appliquer cette composition ainsi qu'un "kit" de teinture.

## Description

La présente invention a pour objet une composition pour la teinture des fibres kératiniques, en particulier des fibres kératiniques humaines telles que les cheveux, contenant, dans un milieu approprié pour la teinture, au moins un précurseur de colorant d'oxydation, au moins une enzyme alcool oxydase, au moins un substrat pour ladite enzyme et au moins un polymère à motif sucre.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance à des composés colorés par un processus de condensation oxydative.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

La coloration est généralement réalisée en milieu fortement alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi la demande de brevet FR 2 769 219 décrit l'utilisation d'une enzyme uricase et de son substrat l'acide urique en coloration d'oxydation pour la teinture de fibres kératiniques. La demande EP-A-0 310 675 décrit l'utilisation de précurseur de colorant d'oxydation de type benzénique en association avec des enzymes telles que la pyranose-oxydase, la glucose-oxydase. Plus récemment, la demande de brevet FR 2 833 492 décrit l'utilisation de l'enzyme alcool-oxydase en tant que seule enzyme dans une composition de coloration d'oxydation pour la teinture de fibres kératiniques.

Le but de la présente invention est de fournir de nouvelles compositions pour la teinture des fibres kératiniques par coloration d'oxydation épaissies et stables, utilisant un système oxydant différent du peroxyde d'hydrogène.

Par "composition épaissie", au sens de la présente demande on entend une composition qui peut être appliquée sur la zone à teindre sans couler hors de cette zone.

De préférence, la viscosité des compositions selon l'invention est supérieure à 200 cp, de manière encore plus préférée supérieure à 500 cp, mesurée à 25°C à l'aide d'un rhéomètre RHEOMAT RM 180 à un taux de cisaillement de 200 s⁻¹.

De manière avantageuse et inattendue, la demanderesse vient maintenant de découvrir qu'il est possible d'atteindre ce but en utilisant au moins un précurseur de colorant d'oxydation, au moins une enzyme alcool oxydase, au moins un substrat pour ladite enzyme et au moins un polymère à motif sucre dans une composition pour la teinture des fibres kératiniques, en particulier des fibres kératiniques humaines telles que les cheveux.

Le polymère à motif sucre peut être associatif ou non.

La composition selon l'invention permet l'obtention de formulations particulièrement homogènes qui, une fois appliquées, respectent la nature des fibres kératiniques et ne présentent pas les problèmes de solubilisation et de cristallisation rencontrés notamment avec le système acide urique/ uricase. La demanderesse a aussi noté que la stabilité de la composition selon l'invention et en particulier la stabilité de l'enzyme alcool oxydase de cette composition est améliorée.

Les compositions selon la présente invention présentent l'avantage de conduire à l'obtention de teintures de couleurs puissantes, peu sélectives et résistantes, ces compositions sont capables d'engendrer des nuances variées de couleur intense et uniforme, sans dégradation significative du cheveu. En outre, on a noté que l'utilisation d'une telle composition améliore la tenue des cheveux permanentés et diminue la porosité du cheveu.

La Demanderesse a également observé que, lors de l'utilisation de la composition selon l'invention, la montée du colorant sur les fibres n'est pas freinée, ce qui est généralement le cas lors de l'emploi de compositions de coloration contenant des épaississants traditionnels, tensio-actifs et solvants.

Or, lorsque la montée du colorant sur les fibres est freinée, l'obtention d'une nuance puissante nécessite alors l'utilisation d'une quantité plus importante de colorant et également d'une quantité plus importante de solvant et/ou de tensio-actif pour solubiliser ce colorant.

D'autres caractéristiques, aspects, objets et avantages de la présente invention apparaîtront encore plus clairement à la lecture de la description et des exemples qui suivent.

Au sens de la présente invention, on entend par "polymère associatif", un polymère possédant dans sa structure au moins une chaîne grasse en C₈-C₃₀ latérale ou terminale. Ces polymères associatifs sont capables en milieu aqueux de s'associer réversiblement entre eux ou avec d'autres molécules tels que des tensioactifs pour conduire à un épaississement accru du milieu.

À titre de polymères à motif sucre utilisables dans les compositions selon l'invention, on peut citer:
- les gommes de guar non-ioniques;
- les gommes de biopolysaccharides d'origine microbienne telles que les gommes de Scléroglucane ou de Xanthane;
- les gommes issues d'exudats végétaux telles que les gommes Arabique, Ghatti, Karaya, Tragacanthe, Carrageenane, Agar et Caroube;
- les pectines;
- les alginates;
- les amidons;
- les dérivés de cellulose choisis parmi les hydroxyalkylcelluloses et carboxyalkylcelluloses, les celluloses cationiques, les hydroxyéthylcelluloses cationiques, les dérivés de cellulose non ioniques, les dérivés de cellulose modifiée.

Par "motif sucre", on désigne au sens de la présente invention, une portion monosaccharidique (i.e. monosaccharide ou oside ou sucre simple) ou une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides (homopolyosides ou hétéropolyosides)]. Les unités saccharidiques peuvent être en outre substituées par des groupements alkyle, ou hydroxyalkyle, ou alcoxy, ou acyloxy, ou carboxyle.

Les gommes de guar non ioniques peuvent être modifiées ou non modifiées.

Les gommes de guar non modifiées sont par exemple les produits vendus sous la dénomination VIDOGUM GH 175 par la société UNIPECTINE et sous la dénomination JAGUAR C par la société MEYHALL.

Selon la présente invention, on préfère utiliser les gommes de guar non-ioniques modifiées par des groupements hydroxyalkyle en C₁-C ₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC 293 et JAGUAR HP 105 par la société RHONE POULENC (MEYHALL) ou sous la dénomination GALACTASOL 4H4FD2 par la société AQUALON.

Les gommes de biopolysaccharides d'origine microbienne telles que les gommes de scléroglucane ou de xanthane, les gommes issues d'exudats végétaux telles que les gomme Arabique, gomme Ghatti, gomme Karaya, Tragacanthe, Carrageenane, Agar et Caroube, les hydroxyalkylcelluloses et les carboxyméthylcelluloses, les pectines, les alginates et les amidons sont bien connus de l'homme de l'art et décrits notamment dans l'ouvrage de Robert L. DAVIDSON intitulé "Handbook of Water soluble gums and resins" édité chez Mc Graw Hill Book Company (1980).

Parmi ces gommes, les scléroglucanes plus particulièrement utilisés selon la présente invention, sont représentés par les produits vendus sous la dénomination ACTIGUM CS par la société SANOFI BIO INDUSTRIES et en particulier ACTIGUM CS 11 et sous la dénomination AMIGEL par la société ALBAN MULLER INTERNATIONAL. D'autres scléroglucanes tels que celui traité au glyoxal dans la demande de brevet français N°2633940 peuvent également être utilisés.

Les gommes de Xanthane plus particulièrement utilisées selon la présente invention, sont représentées par les produits vendus sous les dénominations KELTROL, KELTROL T, KELTROL TF, KELTROL BT, KELTROL RD, KELTROL CG par la société NUTRASWEET KELCO, ou sous les dénominations RHODICARE S, RHODICARE H par la société RHODIA CHIMIE.

Les hydroxyalkylcelluloses sont plus particulièrement des hydroxyéthylcelluloses telles que celles vendues sous les dénominations CELLOSIZE QP3L, CELLOSIZE QP4400H, CELLOSIZE QP30000H, CELLOSIZE HEC30000A, CELLOSIZE POLYMER PCG10, par la société AMERCHOL, ou NATROSOL 250HHR, NATROSOL 250MR, NATROSOL 250M, NATROSOL 250HHXR, NATROSOL 250HHX, NATROSOL 250HR, NATROSOL HX, par la société HERCULES, ou encore TYLOSE H1000 par la société HOECHST.

Les hydroxyalkylcelluloses sont également plus particulièrement des hydroxypropylcelluloses comme les produits vendus sous les dénominations KLUCEL EF, KLUCEL H, KLUCEL LHF, KLUCEL MF, KLUCEL G, par la société AQUALON.

Parmi les carboxyalkylcelluloses, on utilise de préférence la carboxyméthylcellulose dont on peut citer les produits vendus sous les dénominations BLANOSE 7M8/SF, BLANOSE RAFFINEE 7M, BLANOSE 7LF, BLANOSE 7MF, BLANOSE 9M31F, BLANOSE 12M31XP, BLANOSE 12M31P, BLANOSE 9M31XF , BLANOSE 7H, BLANOSE 7M31, BLANOSE 7H3SXF, par la société AQUALON, ou encore AQUASORB A500 et AMBERGUM 1221, par le société HERCULES, ou encore CELLOGEN HP810A et CELLOGEN HP6HS9, par la société MONTELLO, ou encore PRIMELLOSE par la société AVEBE.

Les celluloses cationiques préférées sont les celluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci.

Les hydroxyéthylcelluloses cationiques préférées sont les hydroxyéthylcelluloses cationiques quaternisées modifiées par des groupements comportant au moins une chaîne hydrophobe, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaîne hydrophobe en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.

Les dérivés de cellulose non ioniques peuvent être les hydroxyéthylcelluloses modifiées par des groupements comportant au moins une chaîne hydrophobe tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,

Une autre famille de polymères dérivés de cellulose utilisable est constituée des dérivés de cellulose modifiée par des groupes polyalkylène glycol éther d'alkyl phénol, tel que le produit AMERCELL POLYMER HM-1500® vendu par la société AMERCHOL.

Les polymères à motif sucre sont utilisés dans les compositions selon l'invention de préférence en une quantité pouvant varier d'environ 0,01 à 10% en poids du poids total de la composition de teinture appliquée sur les fibres. Plus préférentiellement, cette quantité varie d'environ 0,1 à 5% en poids.

Dans le cadre de la présente invention, les enzymes alcool oxydases utilisées dans la composition tinctoriale conforme à l'invention appartiennent à la classe E.C.1.1.3 de la nomenclature des enzymes, (voir Enzyme Nomenclature, Academic Press Inc ; 1992).

Lesdites enzymes sont choisies parmi les alcool primaire oxydases ( EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7) encore appelées aryl alcool oxydases.

De préférence l'enzyme utilisée dans la composition selon l'invention est une alcool primaire oxydase ( EC1.1.3.13).

Les enzymes alcool oxydases forment une classe particulière d'enzymes oxydo-réductase à 2 électrons.

L'enzyme alcool oxydase utilisée dans la composition tinctoriale selon l'invention peut être issue d'un extrait de végétaux, d'animaux, de microorganismes (bactérie, champignon, levure, microalgue ou virus), de cellules différenciées ou dédifférenciées, obtenues *in vivo* ou *in vitro,* modifiées ou non modifiées génétiquement, ou synthétiques (obtenues par synthèse chimique ou biotechnologique).

A titre d'exemples, l'enzyme alcool oxydase peut être issue d'une des espèces suivantes : *Rhodococcus erythropolis, Pseudomonas pseudoalcaligenes* qui sont des bactéries, *Aspergillus niger, Kamagataella pastoris, Phanerochaete chrysosporium, Polyporus obtusus, Hansenula polymorpha, Poria contigua, Penicillium simplicissimum, Pleurotus pulmonarius* (champignons), *Pichia sp. (pastoris, methanolica, angusta)* et *Candida sp. (boidinii, albicans, tropicalis)* (levures), *Pinus strobus* qui est une espèce d'origine végétale, *Gastropode mollusc, Manduca sexta* qui sont d'origine animale.

De préférence l'enzyme utilisée dans la composition selon l'invention est une alcool oxydase provenant de *Pichia pastoris*.

Généralement, la concentration en enzyme alcool oxydase utilisée dans la composition tinctoriale est comprise entre 0,05% et 20% en poids, de préférence comprise entre 0,1 et 10% en poids et de manière encore plus préférée entre 0,5 et 8% en poids par rapport au poids de cette composition.

L'activité enzymatique des enzymes alcool oxydases utilisées conformément à l'invention peut être définie à partir de l'oxydation du donneur en condition aérobie. Unité U correspond à la quantité d'enzyme conduisant à la génération de 1 µmole de peroxyde d'hydrogène par minute à un pH donné et à une température de 25°C.

De façon préférentielle la quantité d'alcool oxydase est comprise entre 10³U et 10⁵ U, de préférence entre 2.10³U et 5.10⁴ U pour 100g de composition tinctoriale.

Le ou les substrats pour ladite enzyme sont encore appelés donneurs pour l'enzyme.

Le substrat pour l'enzyme utilisé dans les compositions selon l'invention est de préférence un alcool choisi parmi les alcools primaires, secondaires, les alcools à longue chaîne hydrocarbonée et les alcools aromatiques. Par exemple à titre de donneur pour les alcool primaire oxydases, on peut citer les alcools primaires contenant de 1 à 6 atomes de carbone ; à titre de donneur pour les aryl alcool oxydases : l'alcool benzylique, le 4-terbutyl benzylique alcool, le 3-hydroxy-4- méthoxybenzyl alcool, le vératryl alcool, le 4-méthoxybenzyl alcool, l'alcool cinnamique ; le 2,4 hexadiéne-1-ol peut également être utilisé en tant que donneur pour les aryl alcool oxydases.

Selon une autre variante de l'invention, le substrat pour l'enzyme est un composé portant au moins une fonction alcool aliphatique ou aromatique, approprié pour réagir avec l'enzyme utilisée. Le composé portant au moins une fonction alcool aliphatique ou aromatique peut notamment être un précurseur de colorant d'oxydation ou encore un adjuvant cosmétiquement acceptable par exemple un polymère, un tensioactif, un conservateur porteur d'au moins une fonction alcool. De façon encore plus préférée, le substrat pour l'enzyme est un précurseur de colorant d'oxydation portant au moins une fonction alcool aliphatique ou aromatique. Par exemple, la N-(β-hydroxypropyl)-paraphénylènediamine qui porte une fonction alcool primaire peut avoir la fonction de base d'oxydation et de substrat pour l'alcool oxydase. De même, les coupleurs d'oxydation, tels que le méta- ou le para-aminophénol, peuvent remplir les deux fonctions. De tels précurseurs sont décrits ci-après. Dans cette variante, l'utilisation d'autres substrats de l'enzyme est facultative.

Ainsi la présente demande vise une composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins un polymère à motif sucre, ledit substrat pouvant être substitué (c'est-à-dire remplacé) en tout ou en partie par le précurseur de colorant d'oxydation dans le cas où il porte au moins une fonction alcool aliphatique ou aromatique.

L'utilisation de la composition conforme à l'invention permet de diminuer les risques associés à la manipulation de peroxyde d'hydrogène. De plus, la concentration en conservateurs des compositions selon la présente invention peut être diminuée par l'apport de composés possédant une fonction alcool qui possèdent également des propriétés de conservateur.

Généralement, la concentration du ou des substrats pour l'enzyme est comprise entre 0,01% et 60% en poids par rapport au poids total de la composition et de préférence comprise entre 0,05% et 30% en poids par rapport au poids total de la composition.

Les bases d'oxydation classiques peuvent notamment être choisies parmi les paraphénylènediamines, les bis-phénylalkylènediamines, les para-aminophénols, les ortho-aminophénols et les bases hétérocycliques et leurs sels d'addition.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène, la 3-hydroxy 1-(4' aminophenyl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 2-chloro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino 2,6-dichlorophénol, le 4-amino 6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

Généralement la concentration de la ou des bases d'oxydation est comprise entre 0,0001 et 20%, de préférence entre 0,005 à 6% en poids par rapport au poids total de la composition.

Parmi les coupleurs d'oxydation classiques, on peut notamment citer les métaphénylènediamines, les méta-aminophénols, les métadiphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6-hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition avec une base utilisables dans le cadre de l'invention sont par exemple choisis parmi les sels d'addition avec la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, ces colorants directs additionnels sont choisis parmi les colorants directs cationiques et les colorants directs naturels.

Parmi les colorants directs cationiques utilisables selon l'invention on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition et encore plus préférentiellement de 0,005 à 10% en poids environ.

La composition tinctoriale conforme à l'invention peut également renfermer divers adjuvants additionnels cosmétiquement acceptables, différents des polymères à motifs sucre. Ces agents additionnels sont utilisés classiquement dans les compositions pour la teinture des cheveux, ce sont par exemple des agents antioxydants, des agents de pénétration, des agents séquestrants, des parfums, des tampons, des agents dispersants, des tensioactifs, des agents conditionneurs tels que par exemple des silicones volatiles ou non volatiles, modifiées ou non modifiées, des polymères cationiques, des cations, des agents filmogènes, des céramides, des agents conservateurs, des agents opacifiants, des vitamines ou provitamines.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. Ce solvant peut être le cas échéant un substrat de l'enzyme comme l'éthanol, l'isopropanol. Cela peut être également un composé non substrat de l'enzyme tel que les éthers de polyols comme le 2-butoxyéthanol, le propylèneglycol, le monométhyléther de propylèneglycol, le monoéthyléther et le monométhyléther du diéthylèneglycol, le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 6 et 11 environ, et de préférence entre 7 et 10 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 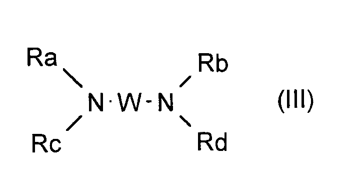 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale peut se présenter sous des formes diverses, telles que sous forme de liquides épaissis, de crèmes, de gels, ou sous toute autre forme appropriée pour réaliser une teinture des fibres kératiniques, et notamment des cheveux humains.

Lorsque les colorants d'oxydation et la ou les alcool oxydases sont présents au sein de la même composition prête à l'emploi, ladite composition est de préférence exempte d'oxygène gazeux de manière à éviter toute oxydation prématurée du ou des colorants d'oxydation.

L'invention a également pour objet un procédé de teinture des fibres kératiniques, et en particulier des fibres kératiniques humaines telles que les cheveux, tel qu'on applique sur ces fibres au moins une composition pour la teinture selon l'invention, la durée de cette application étant la durée suffisante pour développer la coloration désirée.

La couleur est alors révélée par la mise en présence de l'enzyme alcool oxydase et de son substrat en présence d'oxygène.

La composition est appliquée sur les fibres kératiniques. Après un temps de pose de 3 à 60 minutes environ, de préférence 5 à 40 minutes environ, les fibres kératiniques sont rincées, lavées au shampooing, rincées à nouveau puis séchées.

Lorsque la composition pour la teinture est une composition sous une forme prête à l'emploi, elle comprend, dans un milieu approprié pour la teinture des fibres kératiniques, au moins un précurseur de colorant d'oxydation, au moins une enzyme alcool oxydase, au moins un substrat pour ladite enzyme et au moins un polymère à motif sucre, l'ensemble est alors stocké sous forme anaérobie, exempte d'oxygène gazeux.

Selon une variante, ce procédé comporte une étape préliminaire consistant à stocker sous forme séparée, d'une part une composition (A) comprenant, dans un milieu approprié pour la teinture des fibres kératiniques, au moins un précurseur de colorant d'oxydation et d'autre part, une composition (B) renfermant, dans un milieu approprié pour la teinture des fibres kératiniques, au moins une enzyme alcool oxydase, la composition (A) et/ou la composition (B) contenant au moins un substrat pour ladite enzyme, et la composition (A) et/ou la composition (B) contenant au moins un polymère à motif sucre ; puis à procéder au mélange des compositions (A) et (B) au moment de l'emploi avant d'appliquer ce mélange sur les fibres kératiniques.

Selon une variante de l'invention, le procédé comporte une étape préliminaire consistant à stocker sous forme séparée, d'une part une composition (A) comprenant, dans un milieu approprié pour la teinture des fibres kératiniques, au moins un précurseur de colorant d'oxydation, au moins un substrat pour ladite enzyme et au moins un polymère à motif sucre et d'autre part, une composition (B) renfermant, dans un milieu approprié pour la teinture des fibres kératiniques, au moins une enzyme alcool oxydase ; puis à procéder au mélange des compositions (A) et (B) au moment de l'emploi avant d'appliquer ce mélange sur les fibres kératiniques.

La couleur peut être révélée à pH acide, neutre ou alcalin. Dans le cas où le procédé est mis en oeuvre au moyen d'une composition (A) comprenant au moins un précurseur de colorant d'oxydation, au moins un substrat pour ladite enzyme et au moins un polymère à motif sucre et d'une composition (B) renfermant au moins une enzyme alcool oxydase, l'enzyme peut être ajoutée à la composition de l'invention juste au moment de l'emploi ou peut être mise en oeuvre à partir d'une composition la contenant, appliquée simultanément ou séquentiellement à la composition de l'invention.

Cette composition B (dite composition oxydante) peut également renfermer divers adjuvants utilisés classiquement dans les compositions pour la teinture des cheveux et tels que définis précédemment.

Le pH de la composition B dite oxydante est tel qu'après mélange avec la composition tinctoriale A, le pH de la composition résultante appliquée sur les fibres kératiniques varie de préférence entre 6 et 11 environ, et encore plus préférentiellement entre 7 et 10. Il peut être ajusté à la valeur désirée au moyen d'agents acidifiants ou alcalinisants habituellement utilisés en teinture des fibres kératiniques et tels que définis précédemment.

De préférence, l'application de la composition selon l'invention est effectuée à une température comprise entre la température ambiante et 220°C, de préférence entre la température ambiante et 60°C.

Un autre objet de l'invention est un dispositif à plusieurs compartiments ou "kit" de teinture dans lequel un premier compartiment renferme la composition (A) telle que définie ci-dessus et un deuxième compartiment renferme la composition (B) telle que définie ci-dessus. Ce dispositif peut être équipé d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

On prépare les compositions des exemples suivants

| Constituant | Exemple 1 | Exemple 2 | Exemple 3 | Exemple 4 |
|---|---|---|---|---|
| Hydroxyéthylcellulose vendue sous la dénomination NATROSOL 250 HHR par la société AQUALON | 0,2 g | | | |
| Carboxyméthylcellulose vendue sous la dénomination BLANOSE 7M par la société AQUALON | | 0,2 g | | |
| Gomme de Guar vendue sous la dénomination VIDOGUM GH175 par la société UNIPECTINE | | | 0,2 g | |
| Gomme de Scléroglucane vendue sous la dénomination AMIGEL par la société ALBAN MULLER INTERNATIONAL | | | | 0,2 g |
| Ethanol | 25 g | 25 g | 25 g | 25 g |
| paraphénylènediamine | 3.10⁻³ mole | 3.10⁻³ mole | 3.10⁻³ mole | 3.10⁻³ mole |
| métaaminophénol | 3.10⁻³ mole | 3.10⁻³ mole | 3.10⁻³ mole | 3.10⁻³ mole |
| Alcool oxydase | 20000 unités | 20000 unités | 20000 unités | 20000 unités |
| 2-amino-2-méthyl-1-propanol qs | pH 7 | pH 7 | pH 7 | pH 7 |
| Eau distillée qsp. | 100 g | 100 g | 100 g | 100 g |

L'alcool oxydase utilisée est celle commercialisée par la société Biozyme Laboratories sous forme liquide à la concentration de 1980 unités/ml.

L'unité U correspond à la quantité d'enzyme conduisant à la génération de 1 µmole de peroxyde d'hydrogène par minute à pH 7,5 (tampon phosphate 100mM) et à une température de 25°C.

On applique les compositions ci-dessus sur des mèches de cheveux gris naturels et permanentés à 90% de blancs et on laisse poser pendant 30 minutes. Le rapport de bain est fixé à 5. L'alcool oxydase est ajoutée extemporanément. Les cheveux ont ensuite été rincés, lavés avec un shampooing standard, puis séchés.

Les cheveux ont été teints dans des nuances vert kaki.

## Revendications

1. Composition pour la teinture des fibres kératiniques, en particulier des fibres kératiniques humaines telles que les cheveux, contenant, dans un milieu approprié pour la teinture, au moins les composés suivants : un précurseur de colorant d'oxydation ; au moins une enzyme alcool oxydase ; au moins un substrat, portant une fonction alcool, pour ladite enzyme et au moins un polymère à motif sucre, ledit substrat pouvant être substitué en tout ou en partie par le précurseur de colorant d'oxydation dans le cas où ledit précurseur porte au moins une fonction alcool aliphatique ou aromatique.

2. Composition selon la revendication 1 telle que le polymère à motif sucre est associatif.

3. Composition selon la revendication 1 telle que le polymère à motif sucre est non associatif.

4. Composition selon l'une des revendications 1 à 3 telle que le polymère à motif sucre est choisi dans le groupe formé par les gommes de guar non-ioniques, les gommes de biopolysaccharides d'origine microbienne telles que les gommes de Scléroglucane ou de Xanthane, les gommes issues d'exudats végétaux telles que les gommes Arabique, Ghatti, Karaya, Tragacanthe, Carrageenane, Agar et Caroube, les pectines, les alginates, les amidons, les dérivés de cellulose choisis parmi les hydroxyalkylcelluloses et carboxyalkylcelluloses, les celluloses cationiques, les hydroxyéthylcelluloses cationiques, les dérivés de cellulose non ioniques, les dérivés de cellulose modifiée.

5. Composition selon la revendication 4 telle que le polymère à motif sucre de type hydroxyalkylcellulose est une hydroxyéthylcellulose.

6. Composition selon la revendication 4 telle que le polymère à motif sucre de type hydroxyalkylcellulose est une hydroxypropylcellulose.

7. Composition selon la revendication 4 telle que le polymère à motif sucre est une gomme de guar non ionique modifiée par des groupements hydroxyalkyle en C₁-C₆.

8. Composition selon la revendication 7 telle que la gomme de guar non ionique présente un taux d'hydroxyalkylation variant entre 0,4 et 1,2.

9. Composition selon la revendication 4 telle que le polymère à motif sucre de type carboxyalkylcellulose est une carboxyéthylcellulose.

10. Composition selon la revendication 4 telle que le polymère à motif sucre est cationique et est choisi parmi les celluloses et les hydroxyéthylcelluloses quaternisées par un groupement hydrophobe.

11. Composition selon la revendication 4 telle que le polymère à motif sucre est non ionique et est choisi parmi les hydroxyéthylcelluloses modifiées par un groupement hydrophobe.

12. Composition selon l'une quelconque des revendications précédentes telle le polymère à motif sucre est présent en une quantité comprise entre 0,01 et 10%, de préférence entre 0,1 et 5% en poids du poids total de la composition.

13. Composition selon l'une des revendications précédentes telle que l'enzyme alcool oxydase est choisie parmi les alcool primaire oxydases (EC1.1.3.13), les alcool secondaire oxydases (EC 1.1.3.18), les alcool à longue chaîne hydrocarbonée oxydases (EC 1.1.3.20), les alcool polyvinylique oxydases ( EC 1.1.3.30), l'alcool vanillique oxydase (EC 1.1.3.38), les alcool aromatique oxydases (EC 1.1.3.7).

14. Composition selon la revendication 13 telle l'enzyme alcool oxydase est issue d'une des espèces *suivantes* : *Rhodococcus erythropolis, Pseudomonas pseudoalcaligenes, Aspergillus niger, Kamagataella pastoris, Phanerochaete chrysosporium, Polyporus obtusus, Hansenula polymorpha, Poria contigua, Penicillium simplicissimum, Pleurotus pulmonarius, Pichia sp. (pastoris, methanolica, angusta) et Candida sp. (boidinii, albicans, tropicalis), Pinus strobus, Gastropode mollusc, Manduca sexta*, de préférence *Pichia pastoris.*

15. Composition selon l'une des revendications 13 ou 14, telle que la concentration en enzyme alcool oxydase est comprise entre 0,05 et 20% de préférence entre 0,1 et 10% et de manière encore plus préférée entre 0,5 et 8% en poids par rapport au poids total de ladite composition

16. Composition selon l'une des revendications précédentes telle que la quantité d'alcool oxydase est comprise entre 10³U et 10⁵ U, de préférence entre 2.10³U et 5.10⁴U pour 100g de composition tinctoriale.

17. Composition selon l'une des revendications précédentes telle que le substrat pour l'enzyme est un alcool choisi parmi les alcools primaires, secondaires, les alcools à longue chaîne hydrocarbonée et les alcools aromatiques.

18. Composition selon la revendication 17 telle que la concentration du ou des substrats pour l'enzyme est comprise entre 0,01% et 60% en poids par rapport au poids total de la composition et de préférence comprise entre 0,05% et 30% en poids par rapport au poids total de la composition.

19. Composition selon l'une des revendications précédentes telle qu'elle comprend en tant que précurseur de colorant d'oxydation une base d'oxydation choisie parmi les paraphénylènediamines, les bis-phénylalkylènediamines, les para-aminophénols, les ortho-aminophénols, les bases hétérocycliques et leurs sels d'addition.

20. Composition selon la revendication 19 telle que la concentration de la ou des bases d'oxydation est comprise entre 0,0001 et 20% en poids par rapport au poids total de la composition.

21. Composition selon l'une des revendications précédentes telle que la composition comprend en tant que précurseur de colorant d'oxydation un coupleur d'oxydation choisi parmi les métaphénylènediamines, les métaaminophénols, les métadiphénols, les coupleurs naphtaléniques, les coupleurs hétérocycliques ainsi que leurs sels d'addition.

22. Composition selon la revendication 21 telle que la concentration du ou des coupleurs est comprise entre 0,0001 et 20 % en poids par rapport au poids total de la composition.

23. Composition selon l'une des revendications précédentes **caractérisée par le fait qu'**elle renferme un ou plusieurs colorants directs naturels ou cationiques.

24. Procédé de teinture des fibres kératiniques et en particulier des fibres kératiniques humaines telles que les cheveux, **caractérisée par le fait qu'**on applique sur lesdites fibres au moins une composition selon l'une des revendications 1 à 23, pendant une durée suffisante pour développer la coloration désirée.

25. Procédé selon la revendication 24, **caractérisé par le fait que** la composition est une composition prête à l'emploi comprenant, dans un milieu approprié pour la teinture des fibres kératiniques, au moins une composition selon l'une des revendications 1 à 23, l'ensemble étant stocké sous forme anaérobie, exempte d'oxygène gazeux.

26. Procédé selon la revendication 25, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation et d'autre part, une composition (B) renfermant, dans un milieu approprié pour les fibres kératiniques, au moins une enzyme alcool oxydase, la composition (A) et/ou la composition (B) contenant au moins un substrat pour ladite enzyme et la composition (A) et/ou la composition (B) contenant au moins un polymère à motif sucre, puis à procéder au mélange des compositions (A) et (B) au moment de l'emploi avant d'appliquer ce mélange sur les fibres kératiniques.

27. Procédé selon la revendication 26, **caractérisée par le fait qu'**il comporte une étape préliminaire consistant à stocker sous forme séparée, d'une part une composition (A) comprenant, dans un milieu approprié pour la teinture, au moins un précurseur de colorant d'oxydation, au moins un substrat pour ladite enzyme et au moins un polymère à motif sucre et d'autre part, une composition (B) renfermant, dans un milieu approprié pour les fibres kératiniques, au moins une enzyme alcool oxydase, puis à procéder au mélange des compositions (A) et (B) au moment de l'emploi avant d'appliquer ce mélange sur les fibres kératiniques.

28. Dispositif à plusieurs compartiments ou « Kit » de teinture, **caractérisé par le fait qu'**il comporte un premier compartiment renfermant la composition (A) telle que définie à l'une des revendications 26 ou 27 et un second compartiment renfermant la composition (B) telle que définie à l'une des revendications 26 ou 27.
